# EUROPÄISCHE PATENTSCHRIFT

(11) **EP 0 808 293 B1**
(45) Veröffentlichungstag und Bekanntmachung des Hinweises auf die Patenterteilung: **09.01.2002**
(21) Anmeldenummer: 96902932.1
(22) Anmeldetag: 29.01.1996
(51) Int. Cl.: C01G 51/04, H01M 4/52

(54) **VERFAHREN ZUR HERSTELLUNG VON FEINVERTEILTES METALLISCHES KOBALT ENTHALTENDEM KOBALT(II)-OXID**
METHOD OF PRODUCING COBALTOUS OXIDE CONTAINING FINELY-DISPERSED METALLIC COBALT
PROCEDE DE PRODUCTION D'OXYDE COBALTEUX CONTENANT DU COBALT METALLIQUE FINEMENT DISPERSE

(30) Priorität: 10.02.1995 DE 19504319
(43) Veröffentlichungstag der Anmeldung: 26.11.1997
(73) Patentinhaber: H.C. Starck GmbH, 38642 Goslar (DE)
(72) Erfinder: GÖRGE, Astrid, D-38640 Goslar (DE); MEESE-MARKTSCHEFFEL, Juliane, D-38642 Goslar (DE); NAUMANN, Dirk, D-38667 Bad Harzburg (DE); OLBRICH, Armin, D-38723 Seesen (DE); SCHRUMPF, Frank, D-38640 Goslar (DE)
(74) Vertreter: Steiling, Lothar, Dr.
(86) Internationale Anmeldenummer: EP9600336
(87) Internationale Veröffentlichungsnummer: WO9624556

(56) Entgegenhaltungen:
- EP-A- 0 638 520
- DE-A- 2 327 180
- US-A- 3 775 352
- CHEMICAL ABSTRACTS, vol. 96, no. 14, 5.April 1982 Columbus, Ohio, US; abstract no. 114829, KHOKHLACHEVA: "production and thermal decomposition of formates of some nonferrous metals" XP002002751 & DEPOSITED DOC.VINITI, 1980, Seiten 4960-4980,

## Beschreibung

Die vorliegende Erfindung betrifft ein Verfahren zur Herstellung von metallisches Kobalt enthaltendem Kobalt(II)-Oxid sowie dessen Verwendung.

Kobalt(II)-Oxid wird in Mischung mit metallischem Kobalt als Additiv in der positiven Masse von wiederaufladbaren alkalischen Ni-Batterien auf Basis Ni/Cd oder Ni/NiH eingesetzt. Hierzu wird Ni(OH)₂ mit der Kobalt(II)-Oxid/Metallmischung und Hilfsstoffen zu Pasten verarbeitet, welche anschließend in einen elektrisch leitfähigen Elektrodenträger eingearbeitet werden. Die auf diesem Wege hergestellten Elektroden werden durch Trocknen und/oder Sintern weiterverarbeitet, um somit zu Batterien verschiedenster Ausführung zu gelangen.

Für die Herstellung von Knapfzellen werden die elektrochemisch aktiven Elektrodenbestandteile zusammen mit Hilfsstoffen, vornehmlich Graphit oder Nickelpulver, zu Tabletten unterschiedlicher Größe verpresst. Die Anteile des Kobalts in den Elektrodenmassen liegen dabei zwischen 2 bis 10 Gew.-%.

Die Hauptwirkung des Kobaltmetalls beruht laut der EP-A 353 837 darauf, dass während der ersten Ladezyklen (Formierzyklen) das Kobaltmetall entsprechend seines Potentials zunächst zu 2-wertigem Kobalt oxidiert wird und sich dadurch im alkalischen Elektrolyten lösen kann. Die so erhaltenen und eventuell bereits vorliegenden Co²⁺-Ionen diffundieren dann in Richtung der Oberfläche des Nickelhydroxids. Hier werden sie bei weiterer Ladung der Batterie zu Co(III) in Form von CoO(OH) oxidiert. Dieses wiederum bildet sich als Schicht auf der Oberfläche der Nickelhydroxid-Partikel aus und bewirkt bei den folgenden Lade- und Entladezyklen der Batterie die elektrische Leitfähigkeit des Elektrodenmaterials.

Co²⁺-Ionen können aber ebenso in das Schichtgitter des Nickelhydroxides gelangen und dort die Eigenschaften des Hydroxides so modifizieren, dass eine höhere Lade-effizienz des Elektrodenmaterials bewirkt wird. Zusätzlich zu den bereits genannten Eigenschaften kann das in der Elektrodenmasse eingesetzte Kobalt als Sicherheitsreserve bei zu starker Entladung wirken. Hierbei werden Co²⁺-Ionen elektrochemisch wieder reduziert und verhindern somit eine Wasserstoffentwicklung. Kobaltverbindungen mit den oben erwähnten Eigenschaften werden ebenfalls in den Patentschriften US-A 5 032 475 sowie US-A 5 053 292 und der europäischen Patentanmeldung EP-A 523 284 offenbart.

Für die Lade- und Entladezyklen bei der elektrochemischen Oxidation wird das Kobaltmetaltpulver nur bis zu ca. 50 % in der Elektrode nutzbar gemacht, da der überwiegende Teil des Kobalts mit einer stabilen Oxidschicht überzogen ist. Diese Schutzschicht wiederum verhindert die Bildung von Co²⁺-Ionen, die - wie bereits erwähnt - für die Aktivierung der Elektrode notwendig sind. Um diese Schwierigkeit zu umgehen, wurden bislang lösliche Kobaltverbindungen, wie Kobalthydroxid oder -monoxid in die Elektrodenmasse mit eingearbeitet. Dies bewirkte, dass schon vor der elektrochemischen Formierung Co²⁺-Ionen im Elektrolyten gelöst werden und diese sich an der Oberfläche des Nickelhydroxids bereits abscheiden können [Matsumo et al.: The 162^{nd} ECS Fall Meeting Detroit, 18 (1982)].

Nach dem Stand der Technik wird das für die oben beschriebenen Anwendungszwecke verwendete Co(II)-Oxid technisch durch die thermische Zersetzung von Kobaltcarbonat, Kobalthydroxid oder höheren Kobaltoxiden hergestellt. Diese enthalten jedoch - dem thermodynamischen Gleichgewicht folgend - stets einen Überschuss an Sauerstoff und somit Restgehalte von Co(III).

Geringe Spuren von Co(III) im Kobalt(II)-Oxid katalysieren jedoch autokatalytisch die Oxidation des 2-wertigen zu 3-wertigem Kobalt. Dieses bildet jedoch keine im Elektrolyten löslichen Verbindungen, so dass eine Ausbildung der leitfähigen Schicht nach dem oben beschriebenen Mechanismus nicht möglich ist. Hieraus folgt, dass eine hohe Nutzbarkeit der Elektrode nur dann erzielt werden kann, wenn die Co(III)-Anteile so gering wie möglich sind.

Eine Möglichkeit (EP-A 638 520), Kobalt(III)-Anteile zu vermeiden sowie einen geringen Kobaltmetallanteil zu erzeugen, besteht darin, die Calcination der oben beschriebenen Ausgangsstoffe, wie Kobaltcarbonat, Kobalthydroxide und/oder Kobaltoxide, unter Inertgas in Gegenwart entsprechender Mengen Wasserstoff durchzuführen. Dieses Verfahren erfordert jedoch eine aufwendige Prozesskontrolle, d. h. sehr gute Durchmischung und permanente Anpassung der Wasserstoffdosierung bei Durchsatzschwankungen, welche im technischen Maßstab schwer zu vermeiden sind. Nur dann ist eine konstante Produktqualität mit gleichmäßiger Verteilung des metallischen Kobalts gewährleistet.

Aufgabe der vorliegenden Erfindung ist es daher, ein kobaltmetallhaltiges Kobalt(II)oxid bereitzustellen, welches die oben beschriebenen Nachteile nicht aufweist.

Entsprechende Kobalt(II)oxide können erhalten werden durch ein Verfahren zur Herstellung von metallisches Kobalt enthaltendem Kobalt(II)oxid, welches darauf beruht, daß wäßrige Kobaltchlorid und/oder -nitrat und/oder -sulfat-Lösungen mit Alkali- und/oder Erdalkali und/oder Ammonium-carbonat und/oder -hydrogencarbonat und/oder -hydroxid und einer mindestens eine Carboxylgruppe enthaltenden organischen Verbindung umgesetzt werden, wobei ein Kopräzipitat der allgemeinen Formel

Co [(OH)₂]ₐ[O]_{b}[CO₃]_{c}[R]_{d}

erhalten wird, wobei die Summe 1≤ a+b+c+d≤ 1,5 beträgt und R zwei gleiche oder verschiedene Carbonsäurereste darstellt, deren molares Verhältnis d/(a+b+c+d) entsprechend ihrem Reduktionsvermögen eingestellt wird, und das Kopräzipitat nach Abtrennen aus der Lösung calciniert wird. Ein solches Verfahren ist Gegenstand dieser Erfindung.

Es ist allgemein bekannt, daß Kobaltoxalat bei der thermischen Zersetzung in Kobaltmetall und Kohlendioxid zerfällt.

Ähnlich verhält sich auch Weinsäure.

Versaticsäurereste bilden als Zersetzungsprodukte beispielsweise ungesättigte Kohlenwasserstoffe, die ihrerseits reduzierend wirken können. Bei komplizierter aufgebauten Säuren und deren Derivaten muß das Reduktionsvermögen experimentell bestimmt werden. Wichtig ist, daß als organische Säurereste nur solche eingesetzt werden, die keine, bei der Calcination festen Zersetzungsprodukte liefern, um eine Verunreinigung des Produktes mit Kohlenstoff zu vermeiden.

Durch den Einbau der reduzierend wirkenden Anionen in das Kristallgitter des Kopräzipitates ist die reduzierend wirkende Komponente optimal verteilt und man kann zu einer viel gleichmäßigeren Verteilung des metallischen Kobalts gelangen, als dies bei einer Gasphasenreduktion möglich ist.

Bevorzugt können beim erfindungsgemäßen Verfahren als mindestens eine Carboxylgruppe-enthaltende organische Verbindung Carbonsäuren eingesetzt werden. Dabei sind aus der Gruppe der Carbonsäuren insbesondere
- lineare oder verzweigte, gesättigte oder ungesättigte Monocarbonsäuren mit einer Anzahl der C-Atome von 1 bis 9 und/oder
- lineare oder verzweigte, gesättigte oder ungesättigte Polycarbonsäuren mit einer Anzahl der C-Atome von 2 bis 10 und/oder
- cyclische oder heterocyclische, gesättigte oder ungesättigte Mono- und Polycarbonsäuren mit einer Anzahl der C-Atome von 4 bis 14 und/oder
- lineare oder verzweigte, gesättigte oder ungesättigte Mono- und Polyhydroxycarbonsäuren mit einer Anzahl der C-Atome von 2 bis 7 und/oder
- aromatische Hydroxycarbonsäuren mit einer Anzahl der C-Atome von 7 bis 11 und/oder
- cyclische oder aliphatische, gesättigte oder ungesättigte Ketocarbonsäuren mit einer Anzahl der C-Atome von 2 bis 14 geeignet.

Ebenso vorteilhaft können Adipinsäure, Bernsteinsäure, Glutarsäure, Glyoxylsäure, Maleinsäure, Malonsäure, Milchsäure, Oxalsäure, Phthalsäuren, Schleimsäure, Sorbinsäure, Traubensäure, Versaticsäure, Weinsäure und/oder Zitronensäure eingesetzt werden.

In einer weiteren vorteilhaften Ausführungsform des erfindungsgemäßen Verfahrens können auch die Carbonsäuren in teilveresterter Form eingesetzt werden, solange sie noch mindestens eine aktive Carboxylgruppe aufweisen.

Die erfindungsgemäße Umsetzung wird bevorzugt in einem Temperaturbereich von 20°C bis 100°C, vorzugsweise 25°C bis 85°C, durchgeführt.

Es wird angenommen, daß sich zu Beginn der Reduktion in Kristallgitter zunächst einzelne Kobaltatome bilden, welche sich über Diffusionsprozesse zu Clustern und schließlich zu feinen Kobaltausscheidungen vereinigen. Hieraus ergibt sich neben der Konzentration des metallischen Kobalts im Produkt der Einfluss der Calcinationstemperatur auf die Größe der Kobaltpartikel.

Vorzugsweise wird die Calcination in Inertgasatmosphäre bei Temperaturen zwischen 200°C und 1.100°C, besonders bevorzugt 600°C und 1.000°C, durchgeführt. Auf diese Weise können bei moderaten Temperaturen von ca. 600°C metallische Kobaltpartikel in der Größenordnung von <100 nm, bei höheren Calcinationstemperaturen solche im µm-Bereich erhalten werden.

Das gemäß dem erfindungsgemäßen Verfahren erhältliche, feinteiliges metallisches Kobalt enthaltende Kobalt(II)-Oxid weist einen Kobaltgehalt in einer Größenordnung zwischen 79 bis 98 %, vorzugsweise 91 %, auf, wobei die Primärteilchen des im Material enthaltenen Kobaltmetalls homogen verteilt in einer Größenordnung von 50 nm bis 5 µm, vorzugsweise 100 nm bis 1 µm, vorliegen.

Die Korngröße des erfindungsgemäßen Kobaltmetall enthaltenden Kobalt(II)-Oxids kann entsprechend den Anforderungen in den diversen Anwendungen durch verschiedene Parameter, wie z. B. Konzentration, Temperatur, Rührgeschwindigkeit, Reaktionsdauer, kontinuierliche oder diskontinuierliche Fahrweise bei der Fällung, und verschiedene Mahlprozesse vor und/oder nach der Calcination eingestellt werden.

Für die Anwendung in wiederaufladbaren alkalischen Batterien auf Basis Ni/Cd oder Ni/NiH werden in der Regel mittlere Komgrößen der Agglomerate von <3 µm gefordert, um eine homogene Einarbeitung in die Pasten zu ermöglichen und eine hinreichend schnelle Auflösung in alkalischen Elektrolyten (30 % KOH) zu gewährleisten.

Gegenstand dieser Erfindung ist auch die Verwendung der erfindungsgemäßen metallisches Kobalt enthaltenden Kobalt(II)-Oxide als Elektrodenmaterial in elektrochemischen Sekundärzellen.

Im Folgenden wird die Erfindung beispielhaft erläutert, ohne dass hierin eine Einschränkung zu sehen ist.

### Beispiel 1

In einem Rührreaktor wurden 20l Wasser auf 85°C erhitzt. Hierzu wurden gleichzeitig 20 l einer 2 molaren wäßrigen CoCl₂-Lösung und 120 l einer wäßrigen Lösung bestehend aus NaHCO₃ (60 g/l) und Na₂C₂O₄ (5 g/l) zugegeben.

Die hieraus entstandene Suspension wurde 1 h bei 85°C gerührt, filtriert und gewaschen. Das erhaltene Produkt bei T = 85°C bis zur Gewichtskonstanz getrocknet. Man erhält 4,8 kg basisches Kobaltcarbonat/Oxalat, welches bei T = 700°C unter Inertgasatmosphäre calciniert wurde.

Der Co-Gehalt des entstandenen Produktes beträgt 80,3 %.

### Beispiel 2

Eine Vorlage mit 20 l Wasser wurde auf 85°C erhitzt. Hierzu wurden 40 l einer 2 molaren CoCl₂-Lösung und 60 l einer Lösung mit 150 g/l Na₂CO₃ sowie 3 g/l Natriumtartrat synchron zugegeben. Nach erfolgter Reaktion wurde 1 h bei T = 85°C gerührt, heiß abgesaugt und gewaschen. Als Ausbeute wurden 9,8 kg basisches Kobaltcarbonat/Tartrat erhalten, welches der Calcination bei T = 700°C unter Inertgas unterworfen wurde.

Der Co-Gehalt des entstandenen Materials beläuft sich auf 80,0 %.

### Beispiel 3

170 kg NaHCO₃ und 9 kg Weinsäure wurden in 1 m³ Wasser vorgelegt und mit 500 l einer 2 molaren CoCl₂-Lösung bei Raumtemperatur versetzt. Die Reaktionsmischung wurde 1 h bei Raumtemperatur gerührt, filtriert und mit 500 l kaltem Wasser gewaschen. Anschließend wurde der Filterkuchen in 500 l Wasser suspendiert und 2 h auf 85°C erhitzt, filtriert und heiß nachgewaschen. Der getrocknete Filterkuchen (120 kg) wurde bei 700°C der Calcination unter Schutzgas im Drehrohrofen unterworfen. Man erhielt 72 kg Produkt mit einem Cobaltgehalt von 79,9 %.

## Patentansprüche

1. Verfahren zur Herstellung von feinverteiltes Kobaltmetall enthaltendem Kobalt(II)-Oxid mit einem Kobaltgehalt von 79 bis 91 %, **dadurch gekennzeichnet, dass** wäßrige Lösungen von Kobaltsalzen der allgemeinen Formel CoX₂, wobei X = Cl⁻, NO₃⁻, ½ SO₄²⁻ mit Alkali und/oder Erdalkali und/oder Ammonium-carbonat und/oder -hydrogencarbonat und/oder -hydroxid und einer mindestens eine Carboxylgruppe enthaltenden reduzierenden, organischen Verbindung umgesetzt werden, wobei ein Kopräzipitat der allgemeinen Formel
Co[(OH)₂]ₐ[O]_{b}[CO₃]_{c}[R]_{d}
mit 1 ≤ a+b+c+d ≤ 1,5 erhalten wird, wobei R zwei gleiche oder unterschiedliche Carbonsäurereste darstellt, deren molares Verhältnis d/(a+b+c+d) entsprechend dem Reduktionsvermögen des organischen Restes und dem gewünschten Kobaltmetall-Anteil eingestellt und das Kopräzipitat nach Abtrennen aus der Lösung calciniert wird.

2. Verfahren gemäß Anspruch 1, **gekennzeichnet dadurch, dass** als mindestens eine Carboxylgruppe enthaltende organische Verbindung eine oder mehrere Carbonsäuren und/oder deren Salze eingesetzt werden.

3. Verfahren gemäß Anspruch 2, **gekennzeichnet dadurch, dass** als Carbonsäuren lineare oder verzweigte, gesättigte oder ungesättigte Monocarbonsäuren mit einer Anzahl der C-Atome von 1 bis 9 und/oder lineare oder verzweigte, gesättigte oder ungesättigte Polycarbonsäuren mit einer Anzahl der C-Atome von 2 bis 10 und/oder cyclische oder heterocyclische, gesättigte oder ungesättigte Mono- und Polycarbonsäuren mit einer Anzahl der C-Atome von 4 bis 14 und/oder lineare oder verzweigte, gesättigte oder ungesättigte Mono- und/oder Polyhydroxycarbonsäuren mit einer Anzahl der C-Atome von 2 bis 7 und/oder aromatische Hydroxycarbonsäuren mit einer Anzahl der C-Atome von 7 bis 11 und/oder cyclische oder aliphatische, gesättigte oder ungesättigte Ketocarbonsäuren mit einer Anzahl der C-Atome von 2 bis 14 eingesetzt werden.

4. Verfahren gemäß einem der Ansprüche 2 oder 3, **dadurch gekennzeichnet, dass** als Carbonsäuren vorzugsweise Adipinsäure, Bemsteinsäure, Glutarsäure, Glyoxylsäure, Maleinsäure, Malonsäure, Milchsäure, Oxalsäure, Phtalsäuren, Schleimsäure, Sorbinsäure, Traubensäure, Versaticsäure, Weinsäure und/oder Zitronensäure eingesetzt werden.

5. Verfahren gemäß einem oder mehreren der Ansprüche 1 bis 4, **dadurch gekennzeichnet, dass** als mindestens eine Carboxylgruppe enthaltende organische Verbindung die teilveresterten Carbonsäuren gemäß einem oder mehreren der Ansprüche 1 bis 4 eingesetzt werden.

6. Verfahren gemäß einem oder mehreren der Ansprüche 1 bis 5, **dadurch gekennzeichnet, dass** die Umsetzung in einem Temperaturbereich von 20°C bis 100°C, vorzugsweise 25°C bis 85°C, durchgeführt wird.

7. Verfahren gemäß einem oder mehreren der Ansprüche 1 bis 6, **dadurch gekennzeichnet, dass** die Calcination in Inertgasatmosphäre bei Temperaturen zwischen 200°C bis 1.100°C, vorzugsweise 600°C bis 1.000°C, durchgeführt wird.

8. Verwendung von metallisches Kobalt enthaltendem Kobalt(II)-Oxid mit einem Kobaltgehalt zwischen 79 bis 91 %, erhalten gemäß einem oder mehreren der Ansprüche 1 bis 7, wobei die Primärteilchen des im Material enthaltenen Kobaltmetalls homogen verteilt in einer Größenordnung von 50 nm bis 5 µm, vorzugsweise 100 nm bis 1 µm, vorliegen, als Elektrodenmaterial in Sekundärbatterien.

## Claims

1. Process for the production of cobalt(II) oxide containing finely divided cobalt metal and with a cobalt content of 79 to 91%, **characterised in that** aqueous solutions of cobalt salts of the general formula CoX₂, wherein X = Cl⁻, NO₃⁻, ½ SO₄²⁻, are reacted with alkali metal and/or alkaline earth metal and/or ammonium carbonate and/or hydrogen carbonate and/or hydroxide and an organic compound containing at least one carboxyl group, wherein a coprecipitate of the general formula
Co[(OH)₂]ₐ[O]_{b}[CO₃]_{c}[R]_{d}
with 1 ≤ a+b+c+d ≤ 1.5, wherein R denotes two identical or different carboxylic acid residues, the molar ratio of which d/(a+b+c+d) is adjusted in accordance with the reductive capacity of the organic residue and the desired cobalt metal content, and, once separated from the solution, the coprecipitate is calcined.

2. Process according to claim 1, **characterised in that** one or more carboxylic acids and/or the salts thereof are used as the organic compound containing at least one carboxyl group.

3. Process according to claim 2, **characterised in that** the carboxylic acids used are linear or branched, saturated or unsaturated monocarboxylic acids with a number of C atoms from 1-9 and/or linear or branched, saturated or unsaturated polycarboxylic acids with a number of C atoms from 2-10 and/or cyclic or heterocyclic, saturated or unsaturated mono- and polycarboxylic acids with a number of C atoms from 4-14 and/or linear or branched, saturated or unsaturated mono- and/or polyhydroxycarboxylic acids with a number of C atoms from 2-7 and/or aromatic hydroxycarboxylic acids with a number of C atoms from 7-11 and/or cyclic or aliphatic, saturated or unsaturated ketocarboxylic acids with a number of C atoms from 2-14.

4. Process according to one of claims 2 or 3, **characterised in that** the carboxylic acids used are preferably adipic acid, succinic acid, glutaric acid, glyoxylic acid, maleic acid, malonic acid, lactic acid, oxalic acid, phthalic acids, mucic acid, sorbic acid, racemic tartaric acid, versatic acid, tartaric acid and/or citric acid.

5. Process according to one or more of claims 1 to 4, **characterised in that** partially esterified carboxylic acids according to one or more of claims 1 to 4 are used as the organic compound containing at least one carboxyl group.

6. Process according to one or more of claims 1 to 5, **characterised in that** the reaction is performed within a temperature range from 20°C to 100°C, preferably 25°C to 85°C.

7. Process according to one or more of claims 1 to 6, **characterised in that** calcination is performed in an inert gas atmosphere at temperatures of between 200°C and 1100°C, preferably 600°C to 1000°C.

8. Use of cobalt(II) oxide containing metallic cobalt and with a cobalt content of between 79 and 91% obtained according to one or more of claims 1 to 7, wherein the primary particles of the cobalt metal contained in the material are present homogeneously distributed in a size of the order of 50 nm to 5 µm, preferably 100 nm to 1 µm, as an electrode material in secondary batteries.

## Revendications

1. Procédé de préparation d'oxyde de cobalt (II) contenant du cobalt métallique finement divisé avec une teneur en cobalt allant de 79 à 91%, **caractérisé en ce que** des solutions aqueuses de sels de cobalt de formule générale CoX₂, où X = Cl⁻ NO₃⁻, ½ SO₄²⁻ avec des carbonate et/ou hydrogénocarbonate et/ou hydroxyde alcalin et/ou alcalino-terreux et/ou d'ammonium et un composé organique réducteur contenant au moins un radical carboxyle, où l'on obtient un coprécipité de formule générale :
CO[(OH)₂]ₐ[O]_{b}[CO₃]_{c}[R]_{d}
avec 1 ≤ a+b+c+d ≤ 1,5, où R représente deux restes acide carboxylique identiques ou différents, dont le rapport molaire d/(a+b+c+d) correspondant au pouvoir réducteur des restes organiques et la proportion souhaitée de cobalt métallique sont réglés, et le coprécipité est calciné après séparation de la solution.

2. Procédé suivant la revendication 1, **caractérisé en ce que** comme composé organique contenant au moins un radical carboxyle, on met en oeuvre un ou plusieurs acides carboxyliques et/ou leurs sels.

3. Procédé suivant la revendication 2, **caractérisé en ce que** l'on met en oeuvre comme acide carboxylique, les acides monocarboxyliques linéaires ou ramifiés, saturés ou insaturés ayant un nombre d'atomes de carbone allant de 1 à 9, et/ou les acides polycarboxyliques linéaires ou ramifiés, saturés ou insaturés ayant un nombre d'atomes de carbone allant de 2 à 10, et/ou les acides mono- et polycarboxyliques cycliques ou hétérocycliques, saturés ou insaturés ayant un nombre d'atomes de carbone allant de 4 à 14, et/ou les acides mono- et polyhydroxycarboxyliques linéaires ou ramifiés, saturés ou insaturés ayant un nombre d'atomes de carbone allant de 2 à 7, et/ou les acides hydroxycarboxyliques aromatiques ayant un nombre d'atomes de carbone allant de 7 à 11, et/ou les acides cétocarboxyliques cycliques ou aliphatiques, saturés ou insaturés ayant un nombre d'atomes de carbone allant de 2 à 14.

4. Procédé suivant l'une quelconque des revendications 2 ou 3, **caractérisé en ce que** l'on met en oeuvre comme acide carboxylique, de préférence l'acide adipique, l'acide succinique, l'acide glutarique, l'acide glyoxylique, l'acide maléique, l'acide malonique, l'acide lactique, l'acide oxalique, l'acide phtalique, l'acide mucique, l'acide sorbique, l'acide tartrique racémique, l'acide versatique, l'acide tartrique et/ou l'acide citrique.

5. Procédé suivant l'une ou plusieurs des revendications 1 à 4, **caractérisé en ce que** l'on met en oeuvre en tant que composé organique contenant au moins un radical carboxyle, les acides carboxyliques partiellement estérifiés suivant l'une ou plusieurs des revendications 1 à 4.

6. Procédé suivant l'une ou plusieurs des revendications 1 à 5, **caractérisé en ce que** la réaction est réalisée dans la gamme de température allant de 20°C à 100°C, de préférence de 25°C à 85°C.

7. Procédé suivant l'une ou plusieurs des revendications 1 à 6, **caractérisé en ce que** la calcination est réalisée dans une atmosphère de gaz inerte à des températures situées dans l'intervalle allant de 200°C à 1100°C, de préférence de 600°C à 1000°C.

8. Utilisation de l'oxyde de cobalt (II) contenant du cobalt métallique avec une teneur en cobalt allant de 79 à 91%, obtenu suivant une ou plusieurs des revendications 1 à 7, ou les particules primaires du cobalt métallique contenu dans le matériau sont réparties de manière homogène et ont un ordre de grandeur allant de 50 nm à 5 µm, de préférence de 100 nm à 1 µm, comme matériau d'électrode dans des piles secondaires.
